# EUROPEAN PATENT SPECIFICATION

(11) **EP 2 881 130 B1**
(45) Date of publication and mention of the grant of the patent: **06.06.2018**
(21) Application number: 13195756.5
(22) Date of filing: 04.12.2013
(51) Int. Cl.: A61M 5/168

(54) **System for measuring pressure and temperature of a fluid and an apparatus for adjusting or stabilizing the temperature of a patient**
System zur Messung von Druck und Temperatur eines Fluids und Vorrichtung zur Anpassung oder Stabilisierung der Temperatur eines Patienten
Système de mesure de la pression et de la température d'un fluide et appareil permettant de régler ou de stabiliser la température d'un patient

(43) Date of publication of application: 10.06.2015
(73) Proprietor: seiratherm GmbH, 91074 Herzogenaurach (DE)
(72) Inventor: Roth, Matthias, 85376 Giggenhausen (DE); Reichthalhammer, Thomas, 84570 Polling (DE)
(74) Representative: Stellbrink & Partner Patentanwälte mbB

(56) References cited:
- EP-A1- 0 444 462
- WO-A1-2012/084268
- US-A- 4 384 578
- US-B2- 6 889 556

## Description

The disclosed technology relates to a system with a sensor unit for measuring pressure and temperature of a fluid. Moreover, the disclosed technology relates to a tube and a tube assembly for releasable insertion in the sensor unit as well as to an apparatus for adjusting or stabilizing the temperature of a patient.

Document WO 2009/056640 A2 discloses an apparatus and a method for adjusting or stabilizing the body temperature of a patient. The adjustment and stabilization of the body temperature of a patient is achieved by an actively controlled infusion of fluid of a preferably known and/or controlled temperature employing a feedback control with a measured body temperature and a fluid flow being the actuating variable. This document, inter alia, discloses to provide a flexible tube which is branched in at least two proximal tubes and a distal tube such that volume flows of at least two infusion fluids entering the proximal tubes result in a common volume flow in the distal tube. Such system comprises at least one temperature sensor for measuring the temperature in said distal tube and/or at least two temperature sensors for measuring the temperatures of said at least two infusion fluids. WO 2012/084268 A1 discloses another prior art apparatus. The infusion fluid to be infused as well as the tubing has to be sterile. Therefore, the fluid to be infused should not get in contact with non-sterile equipment. In order to avoid any contamination of the infusion fluid, the tubing of infusion devices is often configured as a disposable item for single use and the measuring devices are configured not to get in contact with the fluid.

It is known to mount a temperature sensor on a pipe. Such pipe mounted temperature sensors require time consuming installation of each sensor to the disposable tubing. Accordingly, the frequently occurring replacement of the disposable tubing is time consuming and costly. Moreover, commercially available pipe mounted sensors are only reliable at relatively high flow rates and relatively high heat quantities. In addition, the cabling for the sensors is disturbing not only during installation but sometimes also during operation. Finally, such sensors do not allow a mechanical check whether the sensor is mounted on the pipe or not.

Other systems using water columns involve a complex installation and negatively impact the fluid system. Such systems may have undesired dead volumes.

A third prior art technology makes use of a sterile disposable adapter connected to the top part of a temperature measuring device. The adapter enters the volume flow to be measured. Also this technology requires a relatively time consuming installation of each sensor linked with additional cabling. Moreover, additional sterile adaptors have to be used.

It is known from document US 4,384,578 to provide a bio-medical flow sensor having metal contact shells constructed of thermally conductive metal and curved to conform closely to the curvature of metal tubing segments of a tube. The temperature of the metal segments of the tube and the fluid flow therein is sensed by thermistors. The temperature is measured in order to calculate a flow rate.

Document WO2009/042061 A2 relates to a sensor system using a multi-function sensor head having a slot to accept a tube of deformable material. The head comprises a force sensor mounted on one wall of said slot to contact the outer wall of a tube in said slot and an infrared temperature sensor element that projects a beam of infrared energy into a tube as well as a corresponding infrared light receiver. The infrared temperature sensing is relatively complex and costly. In addition, the correctness of the measured data for the temperature and pressure may be negatively influenced by the positioning of the deformable tube in the slot. If the tube is not positioned straight in the slot, the measurement data may be impacted. For instance, three-dimensional bending may impact the measurements. The tube may also not be centered if bulging occurs. Moreover, particularly thin tubes as frequently used may be prone to bulging. In addition, the set up is susceptible to the tubes being mounted to the sensor in a, e.g., twisted manner, leading to moments resulting in displacement of the tube in the sensor and thus impaired measurements. Also the general shape and material of the tube may affect the accuracy of the measurement. Different tubes may result in different temperatures / pressure levels of the fluid. Reproducibility of measurements and conclusions may thus be affected.
It is an object of the present invention to overcome or ameliorate one or more of the aforementioned drawbacks of the prior art and to provide an improved and/or alternative and/or additional method or device for controlling a temperature of a patient.

It is one aspect of the disclosed technology to measure temperature and pressure of an infusion fluid in a sterile tube wherein the measuring devices do not get into contact with the fluid thereby avoiding any contamination of the fluid. It is a further aspect of the disclosed technology to provide a technology avoiding or reducing the impact of the measuring unit/system on the fluid to be measured.

It is a further aspect of the disclosed technology to provide a sensor unit and a tube (assembly) which is easy to operate/replace, preferably without involving time consuming installation such as cabling or other installations.

It is a further aspect of the disclosed technology to provide a tube being configured as a disposable item, and which is preferably cheap and environmentally friendly.

It is a further aspect of the disclosed technology to provide a technology which is simple and fail safe, preferably with means ensuring correct installation/operation of the unit/system, avoiding inappropriate installation/operation. It is also a preferred aspect to provide a cost effective system design.

It is a further aspect of the disclosed technology to provide a sensing unit being capable to provide reliable temperature/pressure values at low flow rates with a relatively small variation in temperature and/or pressure. It is a further aspect to provide a sensing unit being capable to provide precise and reproducible data, particularly without requiring expensive sensors. It is a further preferred aspect to provide a sensing unit having a quick or relatively quick response time.

Finally, it is a further preferred aspect of the disclosed technology to provide a sensing unit which does not cause excessive heat losses for measuring the pressure and/or temperature.

One or more objects of the present invention are solved by the subject-matter of the independent claim.

The disclosed technology relates to a system preferably comprising a sensor unit for measuring pressure and/or temperature of a fluid. The fluid may be an infusion fluid. The sensor unit comprises a temperature sensor, a pressure sensor, and/or a receiving area or channel for releasable receiving a tube or portion of a tube. The tube of the system conveys or transports the fluid to be measured. The temperature sensor may comprise a temperature sensing portion having a shape that may, preferably substantially, correspond to the outer shape of a first section of the tube. The temperature sensing portion and/or the pressure sensing portion may at least partially define and/or adjoin the receiving area. The receiving area may be configured as a channel. The sensor unit may be configured such that a tube received in the receiving area is positioned to be in proper functional relationship with the pressure sensor and/or the temperature sensor. This configuration leads to a very small, compact and easy to install sensor unit. Preferably, the receiving area/channel extends from one side of the unit to another such that the tube passes through the device, preferably in a straight line. Preferably, the sensor unit, i.e., the temperature sensor, the pressure sensor and/or the receiving area share a common housing.

Having the temperature sensor as well as the pressure sensor located in one unit which releasably receives the tube allows a quick and easy installation of the tube without any additional cabling of each sensor. The number of disturbing cables and/or connectors or other elements is reduced. Both sensors are operatively connected to the tube at the same time or simultaneously without any additional operation. Advantageously, the operator will not incidentally forget to install one of the two sensors thereby reducing the risk of an erroneous set up.

The temperature sensing portion is preferably configured as a first contact portion adapted to at least partially contact the outer shape of the first section of the tube. The pressure sensing portion may be configured as a second contact portion adapted to at least partially contact the outer shape of the second section. The pressure sensor may comprise a pressure sensing/contact portion that may have at least partially a shape that, preferably substantially, corresponds to the outer shape of a second section of the tube. For instance, the pressure sensing/contact portion may be configured as a substantial flat or curved portion, which may correspond to a portion of the receiving area. However, other shapes and/or configurations may also be possible. Preferably, the first contact portion and/or the second contact portion are configured as half shells or at least substantially as half shells. The corresponding shapes, in particular the corresponding contacting portions, allow for increased heat conductivity in the first section, and preferably also for an improved measurement of the displacement of the second section indicative for the change in pressure of the fluid. Corresponding shapes, such as the shape of the outer diameter of a steel sleeve and the inner diameter of the half shell, both preferably made of copper, maximizes the contact surface between sensor and tube. Geometrically, the receiving area preferably has a substantially circular cross section. Also preferably, the first and/or second contact portions have a circle-segment, semi-circular cross-section and/or U-shaped cross section.

Preferably, the masses of the first and/or second contact portion(s) is/are reduced or minimized in order to improve the response time and the sensitivity of the sensor unit, for instance by reducing the size, preferably by reducing the wall thickness of the contact portions. The temperature sensing portion is preferably made of a heat conductive material such as copper. The first contact portion preferably has a thickness of 0,1 mm to 0,75 mm, more preferably of 0,25 mm to 0,5 mm. The half shells may have a round, oval, or polygonal cross sectional shape, which may depend on the corresponding tube.

The first contact portion may be adapted to receive or hold the first section of the tube, preferably in a press fit or interference fit, most preferably without changing or influencing the cross-sectional shape of the tube. Moreover, the second contact portion may (also) be adapted to hold the second section of the tube, preferably in a press fit or interference fit. The tube may by preferably be held from at least two of opposing sides. The tube may then be held without unduly squeezing the tube/changing the cross-sectional shape. Most preferably, the tube has a round shape and is substantially held along the entire perimeter, at least in sections of the tube, preferably at least in the first and/or second sections, most preferably by the half shells. Other clamping or holding technologies may be used such as a snap fit, positive form fit and the like. Preferably, the first contact portion and/or the second contact portion are shaped and/or sized so as to preload the respective first section and/or second section of the tube in the closed position of sensor unit. The preload of the tube caused by the receiving area or channel may vary along the direction of extension of the tube in the housing of the sensor unit. The dimensioning of the housing may cause preferably different and preferably predetermined preloads acting on the different tube sections within the housing. By closing the sensor unit the tube may be pressed into the channel or receiving area.

The sensor unit may comprise a housing with a first or upper housing part. The first housing part comprises a first or upper portion or half of the receiving area. Moreover, the housing may comprise a second or lower housing part. The second housing part may comprise a second or lower portion or half of the receiving area. The tube may be inserted and/or removed in an open position of the first and/or second housing parts. The tube may be held in the first and/or second housing part in a closed position of the first and the second housing parts, preferably in the disclosed interference fit. By closing the sensor housing, the tube may be pressed into the first and second halves of the receiving area or channel. The disclosed technology allows for quick and convenient replacement of the disposable tubing. Only one type and size of tube may be inserted into the housing. Falsified data due to the usage of an inappropriate tube having the wrong shape and/or being made of an inappropriate material may be avoided. In addition, a cross-sectionally fully enclosing housing, at least in the first and/or second portion, advantageously allows applying a defined pressure on the tube, preferably evenly around the entire perimeter. Thus, the positioning of the tube in the channel as well as the pressure applied on the tube during the set up does not negatively influence the measurements.

The housing of the sensor unit may comprise an isolating material such as an isolating resin material further improving the thermal isolation of the fluid. The material of the housing may comprise at least 10vol% air, preferably at least 20vol% air, further preferred at least 30vol% air, and most preferred about 30vol% air. This may particularly improve the sensing quality and reproducibility of the measurement in the preferred set-up.

The sensor housing may be provided with at least one latch at one of the housing parts and with at least one corresponding latch receiving means or recess on the other of the housing parts. The housing may be provided with a pivoting means allowing an opening of the housing in a pivoting movement. Such a configuration preferably allows the housing to be opened with a single hand operation. The tube advantageously may be removed without any further operation. The disclosed technology further improves the ease of use. The housing may be provided with a biasing means configured to hold the two halves in a normally open position. With other words, the housing of the sensor unit needs to be pushed down in order to be closed. The pivoting means may be configured as hinge connecting the first and second housing parts. Preferably, the first and second portions of the sensor unit are substantially collinearly aligned. Moreover, the receiving area or channel may be configured as substantially straight channel with a preferably totally enclosing and preferably round cross-sectional shape, at least in the first and/or second contact portion.

The temperature sensor of the sensor unit may comprise a thermistor connected to the temperature sensing portion. The thermistor may be adapted to derive the temperature of the fluid and/or of the first section of the tube from the temperature of the temperature sensing portion. The temperature of the infusion fluid may be measured by measuring the temperature of the external surface of the first section of the tube. The temperature of the external portion of the first section of the tube is measured by measuring the temperature of the temperature sensing portion, preferably transmitted from the external surface of the first section of the tube by heat conduction. Preferably the temperature sensor comprises at least one half shell made of copper and a thermistor, the half shell preferably having, preferably exactly, the same diameter as the second section of the tube, which is preferably configured as a steel sleeve.

The pressure sensor may comprise a force sensor. The force sector may be connected to a pressure sensing portion. Preferably the force sensor is adapted to generate a signal indicative of the dimensional change of the second section of the tube. The dimensional change is preferably caused by a change in fluid pressure. For instance, an increase of the fluid pressure may cause an expansion of the diameter of the flexible second section, preferably configured as a silicon tube portion. This radial expansion or displacement may be measurable by the force sensor. The disclosed temperature sensor as well as the disclosed pressure sensor measures the temperature and pressure of the infusion fluid without being in contact with the fluid. Therefore, the temperature sensor as well as the pressure sensor avoids any contamination of the infusion fluid. Moreover, the disclosed sensors can be used without any time consuming set up. The tubing used along with the disclosed sensor unit is easy to set up/replace. As already mentioned above, no additional time consuming cabling or the like is required. The construction of the unit comprising temperature sensor as well as of the pressure sensor is relatively simple and reliable.

The sensor unit preferably comprises a closure detecting means adapted to detect whether the housing of the sensor unit is closed or not. Preferably the closure detecting means is adapted to provide this information to a central control unit. The closure detecting means may comprise a hall sensor and a magnet arranged so as to detect whether the first and second housing parts are in the open position or in the closed position. The hall sensor may detect a closed housing if the magnet is located in the vicinity of the hall sensor.

The disclosed closure detecting means is not limited to a sensor unit comprising a temperature sensor and a pressure sensor. With other words, the present technology may also relate to a unit for measuring a temperature of a fluid with a temperature sensor and a receiving area or channel for releasable receiving a tube for conveying the fluid, wherein the temperature sensor comprises a temperature sensing portion having a shape that substantially correspond to the outer shape of a first section of the tube and wherein the sensor unit comprises above closure detecting means. Thus, the pressure sensor is optional.

The provision of the closure detecting of further reduces the risk of operating errors. The sensor unit may be provided with additional or different means for detecting whether the housing is closed and/or the tube is correctly inserted into the sensor unit. That is, the closure detecting means may be configured, for instance, as a latch providing an audible sound upon entry into the recess indicating the closure of the device.

Alternatively or additionally, the pressure sensor may be arranged such that an offset pressure value is at least obtained when the tube is installed/received in the receiving area. The offset value may be obtained when the sensor unit is closed, preferably when the fluid does not flow through the tube. With other words, the pressure sensor provides a value different from zero when the tube is correctly installed in the sensor unit and the fluid does not yet significantly influence the diameter or extension of the tube. This offset value may be obtained by the second contact portion of the sensor unit. The second contact portion may protrude from at least some of the neighboring portions of the receiving area. For instance, the receiving area may have a generally concave, preferably round, cross-sectional shape and at least a portion of the second contact portion protrudes from the cross-sectional concave/round shape radially inwardly. The receiving area at the second contact portion may comprise a recessed portion into which a, preferably generally, flat portion of the second contact portion is inserted such that at least a part of the pressure sensor/force sensor protrudes from a part of the neighboring portions of the receiving area, preferably of the neighboring portions with a concave/round cross-sectional shape. The protruding portion of the second contact portion is preferably adapted to bias the corresponding portion of the second section of the tube, thereby preferably causing above offset pressure, preferably being a predetermined offset value. The protruding portion may cause a predetermined offset value in the force sensor by imposing an predetermined force/pressure on the force sensor, particularly when a tube is received. The pressure sensor is preferably arranged such that an/the offset pressure value is obtained when the housing parts are in the closed position. This allows the measurement of relatively small pressure changes reliably and in a reproducible manner. The receiving area preferably comprises portions of different shapes. The portions of different shapes preferably ensure a correct orientation and/or insertion of the tube. Preferably the temperature sensing portion and the pressure sensing portions have different shapes. The sensor housing may be adapted to be closed only when the tube is correctly inserted in the sensor unit thereby further increasing the correct operation of the device.

The pressure sensor may be connected to a control means adapted to identify a wrong or faulty insertion of the tube based on the signal of the pressure sensor. The sensor unit may be connected to a control means adapted to identify the wrong insertion of the tube or the open position of the first or second housing part based on the signal of the pressure sensor and/or the closing detecting means. Moreover, the controller might be adapted to assume the tubing to be correctly installed and the sensor unit to be closed if the preset offset value is measured by the pressure sensor. Here, the additional closure detecting means might not be required.

The disclosed technology relates to a tube which is releasable insertable in the sensor unit. The tube may comprise a first section and a second section. The first section may be configured as a thin or relatively thin metal sleeve or sheath. The second section may be configured as a resilient or elastic section. The second section may provide a measurable deformation response which may be indicative of a fluid pressure or a change in fluid pressure. Preferably, the deformation response is in the range of 10 micrometers to several hundred micrometers. Preferably the tube is made of different materials such as 1) a material with a good heat conductivity, such as of stainless steel, and most preferably used in the first section, preferably being configured as a metal sleeve, and 2) an elastic or resilient material, preferably silicon, most preferably applied in the second section. The disclosed tubing may be biocompatible and may allow a relatively quick, reliable and/or precise measurement of the temperature and/or pressure without any contact to the sterile fluid. The tube may comprise further sections of different materials, such as PVC being biocompatible and cheap. The second section may be configured as a metal sleeve having a wall thickness of 0,01 mm to 0,5 mm, more preferably between 0,05 mm to 0,35 mm and most preferably between 0,1 mm to 0,2 mm. Such a thin metal sleeve further increase the measurement preciseness at low flow rates of the fluid.

Preferably, the outer shape of the first section of the tube differs from the outer shape of the second section of the tube, at least in portions. The first and second sections may be provided with different dimensions, e.g., different diameters, and/or with different cross sectional shapes.

The disclosed technology also relates to a tube assembly comprising at least two, preferably three of the disclosed tubes. The three tubes preferably are connected via a T-connector-piece. The disclosed technology also relates to a system for measuring pressure and/or temperature of a fluid, preferably of an infusion fluid. The system may comprise at least one of the disclosed sensor units and at least one of the disclosed tubes.

The disclosed technology also relates to an apparatus for adjusting and/or stabilizing the temperature of patient. The apparatus may comprise at least one system preferably with at least one of the disclosed sensor units and/or at least one of the disclosed tubes.

The apparatus may comprise three sensor units adapted to receive three tubes or the tube assembly. The apparatus may be adapted to receive the tubes so that infusion volume flows provided by at least one and preferably two fluid supplies enter two of the three tubes and result in a common volume flow of fluid in the third of the tree tubes. The third tube may be connected to an infusion needle. Two of the three sensor units may be adapted to measure pressure and/or temperature of the volume flows in the two tubes connected to the fluid supplies. Preferably one of the three sensor units is adapted to measure the measure pressure and/or temperature of the volume flow in the third tube connected to the infusion needle. Preferably, the apparatus comprises at least one pump, preferably at least two pumps located at the two tubes connected to the fluid supplies. The at least one pump may be configured as a peristaltic pump. The apparatus may be provided with a controller adapted to control the adjusting and/or stabilizing of the body temperature of patient.

The apparatus may comprise a T-piece fixation means adapted to receive the T-piece connecting the three tubes, preferably by clamping.

Further additional and/or alternative aspects of the disclosed technology are discussed below.
1. System for measuring pressure and temperature of a fluid, comprising at least one sensor unit (10) and at least one releasably insertable tube (T),
   wherein the tube (T) comprises a first section (T1) and a second section (T2),
   wherein the first section (T1) is preferably configured as a metal sleeve, and
   wherein the second section (T2) is preferably a resilient section configured to provide a deformation response indicative of a fluid pressure,
   wherein the sensor unit (10) comprises a temperature sensor (20), a pressure sensor (30), and/or a receiving area (40) for releasably receiving the tube (T) conveying the fluid,
   preferably, wherein the temperature sensor (20) comprises a first contact portion (22) adapted to contact the outer shape of the first section (T1),
   preferably, wherein the pressure sensor (30) comprises a second contact portion (32) adapted to contact the outer shape of the second section (T2), and,
   preferably, wherein the first contact portion (22) and the second contact portion (32) at least partially define and/or adjoin the receiving area (40).
2. The system of aspect 1, wherein the first contact portion (22) and/or the second contact portion (32) are configured as substantially round half shells or sheaths, preferably having a substantially circular, semi-circular and/or U-shape cross section.
3. The system in accordance with any one of the preceding aspects, wherein the first contact portion (22) and/or the second contact portion (32) together with further opposing portions (22', 32') is/are adapted to hold the first section (T1) and the second section (T2) of the tube (T), preferably in a press fit or interference fit, respectively.
4. The system in accordance with any one of the preceding aspects, wherein the sensor unit (10) comprises a housing (50), preferably with a first housing part (52) comprising a first portion/half (40-1) of the receiving area (40) and a second housing part (54) comprising a second portion/half (40-2) of the receiving area (40), and
   preferably wherein the tube (T) may be inserted and/or removed in an open position of the first and second housing parts (52, 54) and/or, preferably, wherein the tube (T) is held by the first and second housing parts (52, 54), preferably by the first and second portion (40-1, 40-2) of the receiving area (40) in a closed position of the first and second housing parts (52, 54).
5. The system in accordance with aspect 4, wherein the first portion (40-1) and the second portion (40-2) of the receiving area (40) form a channel (40) extending substantial straight in the sensor housing (50) and having a round cross sectional shape.
6. The system in accordance with any one of the preceding aspects, wherein at least the first and/or second contact portions (22, 32) together with their corresponding opposing portions (22', 32') totally enclose the first and/or second sections (T1, T2) of the tube (T).
7. The system in accordance with any one of the preceding aspects, wherein at least the first and/or second sections (T1, T2) of the tube (T) are held substantially along the entire perimeter, preferably with a predetermined contact pressure.
8. The system in accordance with any one of the preceding aspects,
   wherein the temperature sensor (20) comprises a thermistor (24) connected to the first contact portion (22), the thermistor (24) preferably being adapted to derive the temperature of the fluid and/or of the first section (T1) from the temperature of the outer surface of the first contact portion (22).
9. The system in accordance with any one of the preceding aspects, wherein the pressure sensor (30) comprises a force sensor (34) connected to the second contact portion (32), the force sensor (34) preferably being adapted to generate a signal indicative for the dimensional change of the second section (T2) caused by a change in fluid pressure, and preferably being adapted to measure an offset value.
10. The system in accordance with any one of the preceding aspects, wherein the sensor unit (10) comprises a closure detecting means adapted to detect whether the housing is closed or not, the closure detecting means preferably comprising a hall sensor and a magnet or a means causing an audible sound upon closure of the housing.
11. The system in accordance with any one of the preceding aspects, wherein the pressure sensor (30) is arranged such that an offset pressure value is at least obtained when the tube (T) is installed in the receiving area (40), the housing (50) is closed, and the fluid does not flow through the tube.
12. The system in accordance with any one of the preceding aspects, wherein the second contact portion (32) comprises a protruding portion (32-1) that protrudes from a neighboring portion of the receiving area (40), wherein the protruding portion (32-1) of the second contact portion (32) is preferably adapted to bias the corresponding portion of the second section (T2) of the tube (T) when the housing parts are in the closed position thereby preferably causing a predetermined offset value in the force sensor (30).
13. The system in accordance with any one of the preceding aspects, wherein the receiving area (40) comprises a recess into which the pressure sensor (30) is inserted so that a generally flat surface of the pressure sensor (30) penetrates the second portion (40-2) of the receiving area (40) thereby forming the protruding portion (32-1).
14. The system in accordance with any one of the preceding aspects, wherein the receiving area (40) comprises portions of different shapes and/or cross-sections so as to ensure a correct orientation/insertion of the tube (T), preferably wherein the first contact portion (22) and the second contact portion (32) have different shapes and/or cross-sections.
14a. The system in accordance with any one of the preceding aspects, wherein the pressure sensor (30) is connected to a control means adapted to identify the wrong insertion of the tube (T) based on the signal of the pressure sensor (30).
14b. Releasably insertable tube (T) for a system in accordance with any one of the preceding aspects, comprising a first section (T1) and a second section (T2), wherein, preferably, the first section (T1) is configured as a metal sleeve, and Wherein, preferably, the second section (T2) is a resilient section configured to provide a measurable deformation response indicative of a fluid pressure, preferably in the range of 10 micrometers to several 100 micrometers.
14c. The system in accordance with any one of the preceding aspects, wherein the outer shape and/or the outer cross-sectional shape of the first section (T1) differs from that of the second section (T2)
14d. The system in accordance with any one of the preceding aspects, comprising a tube assembly with at least two, preferably three tubes (T) in accordance with any one of the preceding claims, preferably wherein the three tubes (T) are connected via a T-connector-piece.
15. Apparatus for adjusting or stabilizing the temperature of a patient by infusion comprising the system in accordance with any one of the preceding aspects, the apparatus preferably comprising one, more preferably two and also preferably at least three sensor units (10-1, 10-2, 10-3),
   wherein the, preferably three, sensor units (10) are adapted to receive, preferably three, tubes (4a, 4b, 4c) or the tube assembly, preferably wherein the apparatus is adapted to receive the three tubes (4a, 4b, 4c) so that volume flows (3a, 3b) of two fluids entering two (4a, 4b) of the three tubes (4a, 4b, 4c) result in a common volume flow (3c) in the third (4c) of the three tubes (4a, 4b, 4c), the third tube (4c) being connected to an infusion needle,
   wherein two (10-1, 10-2) of the preferably three sensor units (10-1, 10-2, 10-3) are adapted to measure pressure and/or temperature of the volume flows (3a, 3b) in the two tubes (4a, 4b) and one (10-3) of the preferably three sensors units (10-1, 10-2, 10-3) is adapted to measure pressure and/or temperature of the volume flow (3c) in the third tube (4c) connected with the infusion needle.

The present invention will become more fully understood from the detailed description given hereinafter and the accompanying drawings which are given by way of illustration only, and thus, are not limitative of the present invention, and wherein:
- Figure 1a: is a perspective drawing of the sensor unit 10 with a tube T received in receiving area 40,
- Figure 1b: is a perspective drawing of a sensor unit 10 without tube T,
- Figure 2: is an exploded view of sensor unit 10, and
- Figure 3: is a schematic drawing of the apparatus for adjusting and/or stabilizing the temperature of the patient.

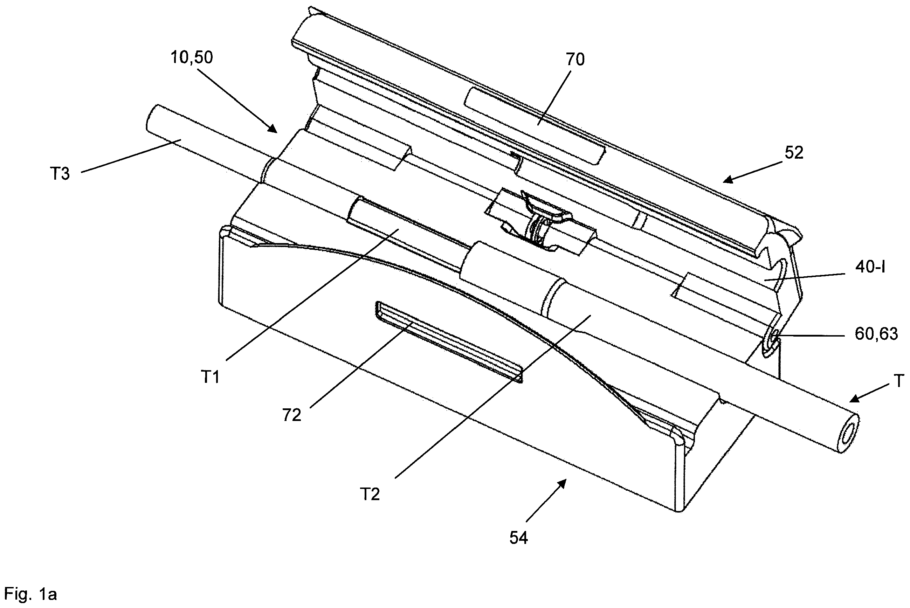

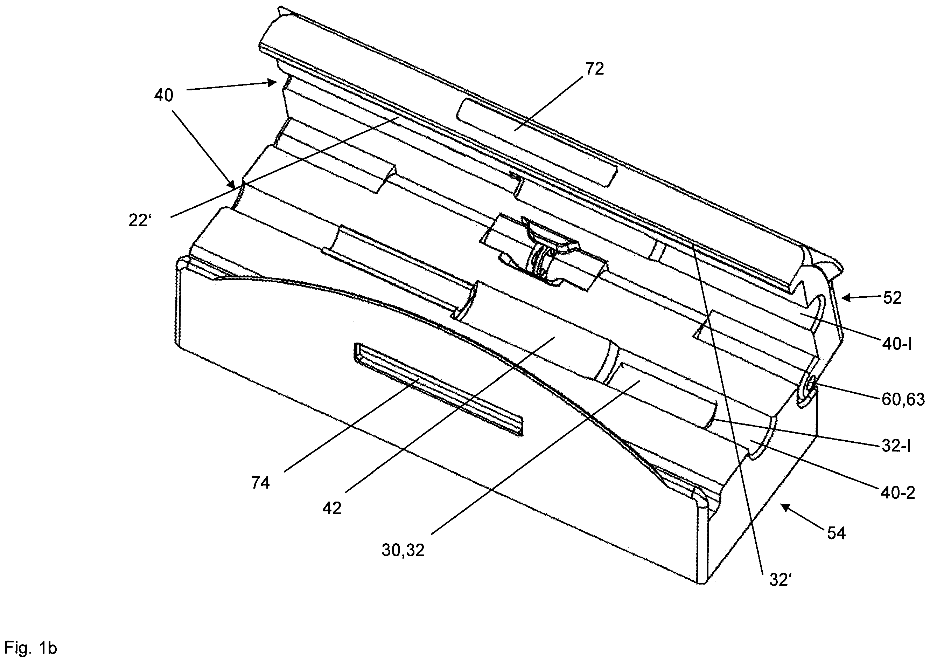

The tube T depicted in Figure 1a comprises three sections. First section T1 is a metal sleeve, preferably having a thin wall thickness of about 0,1 mm to 0,2 mm. First tube section T1 is connected to a second tube section T2 made of a resilient rubber material. Both tube sections T1, T2 have a round cross-sectional shape with different diameters. A third tube section T3 is connected to tube section T1 at the end of the metal sleeve opposite of the end connected to tube section T2. The tube T is configured and received in sensor unit 10 so as to be releasably insertable into sensor unit 10 without any additional tool and with one hand in a single operation. Housing 50 of sensor unit 10 comprises a first housing part 52 and a second housing part 54. The first tube receiving portion 40-1 of receiving area 40 is located in first housing part 52. Tube T is inserted into second tube receiving portion 40-2 (Fig. 1b) of receiving area 40 located in second housing part 45. First housing part 52 is preferably connected to second housing part 54 via hinge 60. First housing part 52 is preferably adapted to pivote relative to second housing part 54 around axis 63 of hinge 60. The front side of upper housing part 52 comprises latch 70 which is adapted to engage with recess 72 located in the front side of housing part 54. When housing 54 is closed, the first and second tube receiving portions 40-1, 40-2, and at least the first and/or second contact portions 22, 32 together with their corresponding opposing portions 22', 32' totally enclose and hold the respective first and second tube portions (T1, T2), preferably with a predetermined contact pressure/force. Here, first contact portion 22 and the corresponding opposing portion 22' located at the receiving portion 40-1 opposite first contact portion 22 together form an enclosure in the closed state of housing 54 having a generally round cross-sectional shape. Similarly second contact portion 32 and opposing second portion 32' form also a generally round cross-sectional shape. This configuration increases the reproducibility of the measurements.

Figure 1b depicts sensor units 10 without tube T. Second half 40-2 of receiving area 40 is located in the second housing part 54. First portion 40-1 and second portion 50-2 together constitute receiving area 40. First portion 40-1 and second portion 40-2 here have a substantially round cross-sectional shape. Thus, first portion 40-1 and second portion 40-2 together form a substantially round channel 40 for tube T when housing 50 is closed. First portion 40-1 and second portion 40-2 comprise different sections of different diameter. Temperature sensor 20 comprises a first contact portion or temperature sensing portion 22 (hereafter, only the term first contact portion is used for the temperature sensing portion) which is here configured as a half shell or sheath 22 located in second portion 40-2. It is also possible to provide temperature sensing portion 22 with an additional second half shell 22' in first receiving portion 40-1 of receiving area 40. Temperature sensing portion 22 defines a part of second part 40-2. Here, half shell 22 is fixed to the inner wall of curved second portion 40-2. Second portion 40-2 of receiving area 40 comprises a recessed portion or recess in which pressure sensing portion 32 of pressure sensor 30 is received/located. Pressure sensing portion 32 also define at least partially second portion 40-2 of receiving area 40. This pressure sensing portion 32 defining second portion 40-2 is here configured as a substantial flat portion which protrudes from the neighboring portions of the second portion 40-2. The flat portion of pressure sensing portion 32 is a portion of the force/pressure sensor 30 inserted into the recess. The difference in heights of the protruding pressure sensing portion or second contact portion 32 is indicated with reference numeral 32-1. The protruding second contact portion 32-1 biases tube T at tube portion T2 when housing 50 is closed causing a predetermined offset value in the force sensor 30 by imposing an predetermined force/pressure on the force sensor 30. This causes pressure sensor 30 to detect an offset pressure value indicating that tube T is inserted into sensing unit 10. The particular design with the recess, the protrusion 32-1 and the concave cross-sectional neighboring portions of the receiving portions 40-2 enables the use of force sensors 34 having a simple, cost effective and robust design. The contact surface of the pressure/force sensor 30, 34 in contact with tube T is relatively large. The measuring results from such sensors may be more precise. Given the preset and reproducible geometrical relationships between tube T and the second contact portion 32 when housing 50 is in the closed position reproducible and thus reliable data can be obtained.

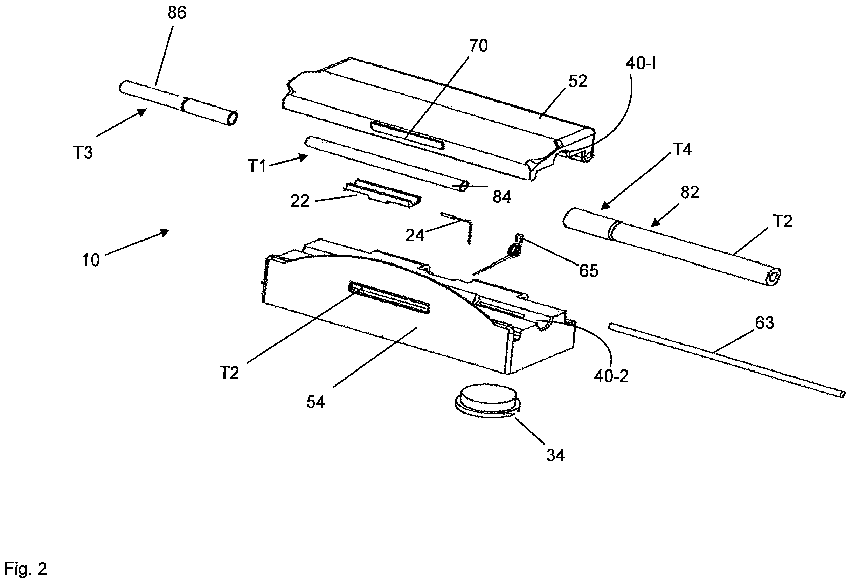

Inbetween temperature sensing portion 22 and pressure sensing portion 32 may be located a connecting portion 42 having an increased diameter relative to the diameter of the temperature sensing portion 22 and/or pressure sensing portion 32. This portion 42 receives section T4 (Fig. 2) of tube T in which the, preferably silicone, tube 82 is received by metal sleeve 84. Portion 42 additionally fixes tube T in sensor unit 10 in a lateral direction. When tube T inserted into sensor 10 and housing 50 is closed, first section T1 is received in first contact portion 22 with a press fit. Moreover, the second section T2 of tube T is also received in second contact portion 32 with a press fit. The different diameters of the different sections T1, T2, T3, T4 (Fig. 2) and of the different receiving portions 22, 32, of the sensor unit 10 may assist in ensuring the correct insertion of the tube in housing 50.

Figure 2 depicts an exploded view of sensor unit 10. Tube T consists of, preferably at least, three parts 82, 84, 86 divided in four sections T1 to T4. Part 82 preferably is a silicone tube comprising second section T2 and fourth section T4. Metal tube or metal sleeve 84 comprises the first section T1 located here in the middle of sleeve 84. PVC tube 86 comprises the third section T3. Thermistor 24 is connected with temperature sensing portion 22 adapted to receive first section T1. Sensor 34 is configured as a generally flat and generally round sensor. The generally flat top surface of force sensor 34 is inserted from the exterior, here form below, into the recessed portion of second portion 40-2 of channel 40. Resilient biasing means or spring 65 forms here part of hinge 60 and is adapted to hold housing 50 in a normally open position. Not shown here is a magnet which may be located in first housing part 52 as well as a hall sensor which may be located in second housing part 54.

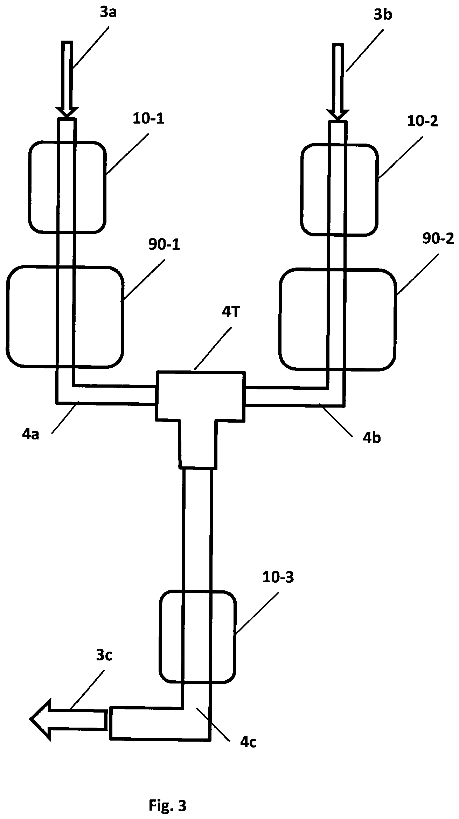

Figure 3 is schematically showing the disclosed apparatus for adjusting or stabilizing the temperature of a patient by infusion. The apparatus comprises three sensor units 10-1, 10-2 and 10-3 into which a tube assembly of three tubes 4a, 4b, 4c is received. Replacement of the tubes does not require any additional electrical cabling. The three tubes 4a, 4b, 4c are connected to each other via T-piece 4T. The tubes 4a, 4b, 4c are received in sensor units 10-1, 10-2, 10-3 in a press fit so that preferably no further mechanical fixation is required in this embodiment. Moreover, T-piece 4T is preferably received in a holding device which may also be adapted to clamp T-piece 4T. The holding device may be provided with LEDs indicating with different colors, preferably different lights which of the tubes 4a, 4b provides a volume flow of infusion fluid with a relatively high temperature and which tube of the tubes 4a, 4b provides a volume flow with a relatively low fluid temperature. Peristaltic pumps 90-1, 90-2 are located so as to receive the first tubing 4a or the second tubing 4b and are adapted to adjust the volume flows 3a and 3b, respectively. Also not depicted in Figure 3 is the control unit adapted to control the fluid temperature and the amount of fluid to be infused, i.e. the volume flow 3c thereby adjusting or stabilizing the temperature of a patient.

While the disclosure has been illustrated and described in detail in the drawings and foregoing description, such illustration and description are to be considered illustrative or exemplary and non-restrictive; the disclosure is thus not limited to the disclosed embodiments. Variations to the disclosed embodiments can be understood and effected by those skilled in the art and practicing the claimed disclosure, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures can not be used to advantage. The present technology is also understood to encompass the exact terms, features, numerical values or ranges etc., if in here such terms, features, numerical values or ranges etc. are referred to in connection with terms such as "about, ca., substantially, generally, at least" etc. In other words, "about 3" shall also comprise "3" or "substantially perpendicular" shall also comprise "perpendicular". Any reference signs in the claims should not be considered as limiting the scope.

## Claims

1. System for measuring pressure and temperature of a fluid, comprising at least one sensor unit (10) and at least one releasably insertable tube (T), wherein the tube (T) comprises a first section (T1) and a second section (T2),
wherein the first section (T1) is configured as a metal sleeve, and
wherein the second section (T2) is a resilient section configured to provide
a deformation response indicative of a fluid pressure,
wherein the sensor unit (10) comprises a temperature sensor (20), a pressure sensor (30), and a receiving area (40) for releasably receiving the tube (T) conveying the fluid,
wherein the temperature sensor (20) comprises a first contact portion (22) adapted to contact the outer shape of the first section (T1),
wherein the pressure sensor (30) comprises a second contact portion (32) adapted to contact the outer shape of the second section (T2), and
wherein the first contact portion (22) and the second contact portion (32) at least partially define and/or adjoin the receiving area (40).

2. The system of claim 1, wherein the first contact portion (22) and/or the second contact portion (32) are configured as substantially round half shells.

3. The system in accordance with any one of the preceding claims, wherein the first contact portion (22) and/or the second contact portion (32) together with further opposing portions (22', 32') is/are adapted to hold the first section (T1) and the second section (T2) of the tube (T) in a press fit or interference fit, respectively.

4. The system in accordance with any one of the preceding claims, wherein the sensor unit (10) comprises a housing (50) with a first housing part (52) comprising a first portion/half (40-1) of the receiving area (40) and a second housing part (54) comprising a second portion/half (40-2) of the receiving area (40), and
wherein the tube (T) may be inserted and/or removed in an open position of the first and second housing parts (52, 54) and wherein the tube (T) is held by the first and second housing parts (52, 54), preferably by the first and second portion (40-1, 40-2) of the receiving area (40) in a closed position of the first and second housing parts (52, 54).

5. The system in accordance with any one of the preceding claims, wherein at least the first and/or second sections (T1, T2) of the tube (T) are held substantially along the entire perimeter, preferably with a predetermined contact pressure.

6. The system in accordance with any one of the preceding claims,
wherein the temperature sensor (20) comprises a thermistor (24) connected to the first contact portion (22), the thermistor (24) being adapted to derive the temperature of the fluid and/or of the first section (T1) from the temperature of the outer surface of the first contact portion (22).

7. The system in accordance with any one of the preceding claims, wherein the pressure sensor (30) comprises a force sensor (34) connected to the second contact portion (32), the force sensor (34) being adapted to generate a signal indicative for the dimensional change of the second section (T2) caused by a change in fluid pressure, and being adapted to measure an offset value.

8. The system in accordance with any one of the preceding claims, wherein the sensor unit (10) comprises a closure detecting means adapted to detect whether the housing is closed or not, the closure detecting means preferably comprising a hall sensor and a magnet or a means causing an audible sound upon closure of the housing.

9. The system in accordance with any one of the preceding claims, wherein the pressure sensor (30) is arranged such that an offset pressure value is at least obtained when the tube (T) is installed in the receiving area (40), the housing (50) is closed, and the fluid does not flow through the tube.

10. The system in accordance with any one of the preceding claims, wherein the second contact portion (32) comprises a protruding portion (32-1) that protrudes from a neighboring portion of the receiving area (40), wherein the protruding portion (32-1) of the second contact portion (32) is adapted to bias the corresponding portion of the second section (T2) of the tube (T) when the housing parts are in the closed position thereby causing a predetermined offset value in the force sensor (30).

11. The system in accordance with any one of the preceding claims, wherein the receiving area (40) comprises a recess into which the pressure sensor (30) is inserted so that a generally flat surface of the pressure sensor (30) penetrates the second portion (40-2) of the receiving area (40) thereby forming the protruding portion (32-1).

12. The system in accordance with any one of the preceding claims, wherein the receiving area (40) comprises portions of different shapes so as to ensure a correct orientation/insertion of the tube (T), preferably wherein the first contact portion (22) and the second contact portion (32) have different shapes.

13. The system in accordance with any one of the preceding claims, wherein the pressure sensor (30) is connected to a control means adapted to identify the wrong insertion of the tube (T) based on the signal of the pressure sensor (30).

14. Releasably insertable tube (T) for a system in accordance with any one of the preceding claims, comprising a first section (T1) and a second section (T2),
wherein the first section (T1) is configured as a metal sleeve, and
wherein second section (T2) is a resilient section configured to provide deformation response indicative of a fluid pressure, preferably in the range of 10 micrometers to several 100 micrometers.

15. Apparatus for adjusting or stabilizing the temperature of a patient by infusion comprising the system in accordance with any one of the preceding claims, the apparatus comprising at least three sensor units (10-1, 10-2, 10-3),
wherein the three sensor units (10) are adapted to receive three tubes (4a, 4b, 4c), wherein the apparatus is adapted to receive the three tubes (4a, 4b, 4c) so that volume flows (3a, 3b) of two fluids entering two (4a, 4b) of the three tubes (4a, 4b, 4c) result in a common volume flow (3c) in the third (4c) of the three tubes (4a, 4b, 4c), the third tube (4c) being connected to an infusion needle,
wherein two (10-1, 10-2) of the three sensor units (10-1, 10-2, 10-3) are adapted to measure pressure and temperature of the volume flows (3a, 3b) in the two tubes (4a, 4b) and one (10-3) of the three sensors units (10-1, 10-2, 10-3) is adapted to measure pressure and temperature of the volume flow (3c) in the third tube (4c) connected with the infusion needle.

## Patentansprüche

1. System zum Messen von Druck und Temperatur eines Fluids (Flüssigkeiten und Gase), umfassend mindestens eine Sensoreinheit (10) und mindestens ein lösbar einsetzbares Rohr (T),
wobei das Rohr (T) einen ersten Teilbereich (T1) und einen zweiten Teilbereich (T2) umfasst,
wobei der erste Teilbereich (T1) als Metallhülse konfiguriert ist, und
wobei der zweite Teilbereich (T2) ein elastischer Teilbereich ist, der dazu konfiguriert ist, eine Deformationsreaktion bereitzustellen, die auf einen Druck eines Fluids hinweist,
wobei die Sensoreinheit (10) einen Temperatursensor (20), einen Druckfühler (30) und einen Aufnahmebereich (40) zur lösbaren Aufnahme des das Fluid transportierenden Rohrs (T) umfasst,
wobei der Temperaturfühler (20) einen ersten Kontaktabschnitt (22) umfasst, der dazu geeignet ist, die äußere Form des ersten Teilbereichs (T1) zu berühren,
wobei der Druckfühler (30) einen zweiten Kontaktabschnitt (32) umfasst, der dazu geeignet ist, die äußere Form des zweiten Teilbereichs (T2) zu berühren, und
wobei der erste Kontaktabschnitt (22) und der zweite Kontaktabschnitt (32) den Aufnahmebereich (40) mindestens teilweise definieren und/oder daran angrenzen.

2. System nach Anspruch 1, wobei der erste Kontaktabschnitt (22) und/oder der zweite Kontaktabschnitt (32) als im Wesentlichen runde Halbschalen konfiguriert ist/sind.

3. System nach einem der vorstehenden Ansprüche, wobei der erste Kontaktabschnitt (22) und/oder der zweite Kontaktabschnitt (32) zusammen mit weiteren gegenüberliegenden Abschnitten (22', 32') dazu geeignet ist/sind, den ersten Teilbereich (T1) und den zweiten Teilbereich (T2) des Rohrs (T) in Press- oder Übermaßpassung zu halten.

4. System nach einem der vorstehenden Ansprüche, wobei die Sensoreinheit (10) ein Gehäuse (50) mit einem ersten Gehäuseteil (52) umfasst, der eine/n erste/n Abschnitt/Hälfte (40-1) des Aufnahmebereichs (40) umfasst, und einen zweiten Gehäuseteil (54), der eine/n zweite/n Abschnitt/Hälfte (40-2) des Aufnahmebereichs (40) umfasst, und
wobei das Rohr (T) in einer offenen Position der ersten und zweiten Gehäuseteile (52, 54) eingesetzt und/oder entfernt werden kann, und wobei das Rohr (T) durch die ersten und zweiten Gehäuseteile (52, 54), vorzugsweise durch den ersten und zweiten Abschnitt (40-1, 40- 2) des Aufnahmebereichs (40), in einer geschlossenen Position der ersten und zweiten Gehäuseteile (52, 54) gehalten wird.

5. System nach einem der vorstehenden Ansprüche, wobei mindestens der erste und/oder zweite Teilbereich (T1, T2) des Rohrs (T) im Wesentlichen entlang des gesamten Umfangs, vorzugsweise bei vorgegebenem Kontaktdruck, gehalten wird.

6. System nach einem der vorstehenden Ansprüche,
wobei der Temperaturfühler (20) einen Thermistor (24) umfasst, der mit dem ersten Kontaktabschnitt (22) verbunden ist, wobei der Thermistor (24) dazu geeignet ist, die Temperatur des Fluids und/oder des ersten Teilbereichs (T1) von der Temperatur der äußeren Fläche des ersten Kontaktabschnitts (22) abzuleiten.

7. System nach einem der vorstehenden Ansprüche, wobei der Druckfühler (30) einen Kraftaufnehmer (34) umfasst, der mit dem zweiten Kontaktabschnitt (32) verbunden ist, wobei der Kraftaufnehmer (34) dazu geeignet ist, ein Signal zu generieren, das die Maßänderung des zweiten Teilbereichs (T2) angibt, die durch eine Änderung des Drucks des fluids entsteht, und dazu angepasst ist, einen Korrekturwert zu messen.

8. System nach einem der vorstehenden Ansprüche, wobei die Sensoreinheit (10) ein Mittel zur Erfassung des geschlossenen Zustands umfasst, das dazu geeignet ist, zu erfassen, ob das Gehäuse geschlossen ist oder nicht, wobei das Mittel zur Erfassung des geschlossenen Zustands vorzugsweise einen Hall-Sensor und einen Magneten oder ein Mittel, das beim Schließen des Gehäuses ein hörbares Geräusch verursacht, umfasst.

9. System nach einem der vorstehenden Ansprüche, wobei der Druckfühler (30) so angeordnet ist, dass ein Korrektur-Druckwert mindestens erhalten wird, wenn das Rohr (T) im Aufnahmebereich (40) eingebaut ist, das Gehäuse (50) geschlossen ist und das Fluid nicht durch das Rohr strömt.

10. System nach einem der vorstehenden Ansprüche, wobei der zweite Kontaktabschnitt (32) einen überstehenden Abschnitt (32-1) umfasst, der von einem angrenzenden Abschnitt des Aufnahmebereichs (40) übersteht, wobei der überstehende Abschnitt (32- 1) des zweiten Kontaktabschnitts (32) dazu geeignet ist, den entsprechenden Abschnitt des zweiten Teilbereichs (T2) des Rohrs (T) vorzubelasten, wenn die Gehäuseteile in geschlossener Position sind, und dabei einen vorgegebenen Korrekturwert im Kraftaufnehmer (30) zu verursachen.

11. System nach einem der vorstehenden Ansprüche, wobei der Aufnahmebereich (40) eine Vertiefung umfasst, in die der Druckfühler (30) eingesetzt wird, so dass eine allgemein glatte Fläche des Druckfühlers (30) durch den zweiten Abschnitt (40-2) des Aufnahmebereichs (40) dringt und dabei den überstehenden Abschnitt (32-1) bildet.

12. System nach einem der vorstehenden Ansprüche, wobei der Aufnahmebereich (40) Abschnitte von unterschiedlichen Formen umfasst, um eine richtige Ausrichtung/Einsetzung des Rohrs (T) zu gewährleisten, wobei vorzugsweise der erste Kontaktabschnitt (22) und der zweite Kontaktabschnitt (32) unterschiedliche Formen aufweisen.

13. System nach einem der vorstehenden Ansprüche, wobei der Druckfühler (30) mit einem Steuerungsmittel verbunden ist, das dazu angepasst ist, falsches Einsetzen des Rohrs (T) basierend auf dem Signal des Druckfühlers (30) zu erkennen.

14. Lösbar einsetzbares Rohr (T) für ein System nach einem der vorstehenden Ansprüche, das einen ersten Teilbereich (T1) und einen zweiten Teilbereich (T2) umfasst, wobei der erste Teilbereich (T1) als Metallhülse konfiguriert ist, und wobei der zweite Teilbereich (T2) ein elastischer Teilbereich ist, der dazu konfiguriert ist, eine Deformationsreaktion bereitzustellen, die einen Druck eines Fluids anzeigt, vorzugsweise im Bereich von 10 Mikrometer bis mehrere 100 Mikrometer.

15. Vorrichtung zum Einstellen oder Stabilisieren der Temperatur eines Patienten durch Infusion, umfassend das System nach einem der vorstehenden Ansprüche, wobei die Vorrichtung mindestens drei Sensoreinheiten (10-1, 10-2, 10-3) umfasst,
wobei die drei Sensoreinheiten (10) dazu angepasst sind, drei Rohre (4a, 4b, 4c) aufzunehmen, wobei die Vorrichtung dazu geeignet ist, die drei Rohre (4a, 4b, 4c) aufzunehmen, so dass Volumenströme (3a, 3b) von zwei Fluids, die in zwei (4a, 4b) der drei Rohre (4a, 4b, 4c) eintreten, einen gemeinsamen Volumenstrom (3c) im dritten (4c) der drei Rohre (4a, 4b, 4c) ergeben, wobei das dritte Rohr (4c) mit einer Infusionsnadel verbunden ist,
wobei zwei (10-1, 10-2) der drei Sensoreinheiten (10-1, 10-2, 10-3) dazu geeignet sind, den Druck und die Temperatur der Volumenströme (3a, 3b) in den beiden Rohren (4a, 4b) zu messen, und eine (10-3) der drei Sensoreinheiten (10-1, 10-2, 10-3) dazu angepasst ist, den Druck und die Temperatur des Volumenstroms (3c) im dritten mit der Infusionsnadel verbundenen Rohr (4c) zu messen.

## Revendications

1. Système de mesure de la pression et de la température d'un fluide, comprenant au moins une unité de détection (10) et au moins un tube insérable de manière amovible (T),
dans lequel le tube (T) comprend une première section (T1) et une deuxième section (T2),
dans lequel la première section (T1) est configurée en tant que manchon métallique, et
dans lequel la deuxième section (T2) est une section élastique configurée pour fournir une réponse de déformation indiquant une pression de fluide,
dans lequel l'unité de détection (10) comprend un capteur de température (20), un capteur de pression (30), et une zone de réception (40) pour la réception amovible du tube (T) transportant le fluide,
dans lequel le capteur de température (20) comprend une première portion de contact (22) adaptée pour entrer en contact avec la forme extérieure de la première section (T1),
dans lequel le capteur de pression (30) comprend une deuxième portion de contact (32) adaptée pour entrer en contact avec la forme extérieure de la deuxième section (T2), et
dans lequel la première portion de contact (22) et la deuxième portion de contact (32) définissent et/ou sont contiguës à la zone de réception (40) au moins partiellement.

2. Système selon la revendication 1, dans lequel la première portion de contact (22) et/ou la deuxième portion de contact (32) sont configurées en tant que demi-coques sensiblement rondes.

3. Système selon l'une quelconque des revendications précédentes, dans lequel la première portion de contact (22) et/ou la deuxième portion de contact (32) conjointement avec d'autres portions opposées (22', 32') est/sont adaptée/s pour retenir la première section (T1) et la deuxième section (T2) du tube (T) dans un ajustement serré ou ajustement d'interférence, respectivement.

4. Système selon l'une quelconque des revendications précédentes, dans lequel l'unité de détection (10) comprend un logement (50) avec une première partie de logement (52) comprenant une première portion/moitié (40-1) de la zone de réception (40) et une deuxième partie de logement (54) comprenant une deuxième portion/moitié (40-2) de la zone de réception (40), et
dans lequel le tube (T) peut être inséré et/ou retiré dans une position ouverte des première et deuxième parties de logement (52, 54) et dans lequel le tube (T) est retenu par les première et deuxième parties de logement (52, 54), de préférence par la première et deuxième portion (40-1, 40-2) de la zone de réception (40) dans une position fermée des première et deuxième parties de logement (52, 54).

5. Système selon l'une quelconque des revendications précédentes, dans lequel au moins les première et/ou deuxième sections (T1, T2) du tube (T) sont retenues sensiblement le long de l'ensemble du périmètre, de préférence avec une pression de contact prédéterminée.

6. Système selon l'une quelconque des revendications précédentes,
dans lequel le capteur de température (20) comprend une thermistance (24) reliée à la première portion de contact (22), la thermistance (24) étant adaptée pour déduire la température du fluide et/ou de la première section (T1) à partir de la température de la surface extérieure de la première portion de contact (22).

7. Système selon l'une quelconque des revendications précédentes, dans lequel le capteur de pression (30) comprend un capteur de force (34) relié à la deuxième portion de contact (32), le capteur de force (34) étant adapté pour générer un signal indiquant le changement dimensionnel de la deuxième section (T2) causé par un changement de pression de fluide, et étant adapté pour mesurer une valeur de décalage.

8. Système selon l'une quelconque des revendications précédentes, dans lequel l'unité de détection (10) comprend un moyen de détection de fermeture adapté pour détecter si le logement est fermé ou non, le moyen de détection de fermeture comprenant de préférence un capteur à effet Hall et un aimant ou un moyen causant un son audible lors de la fermeture du logement.

9. Système selon l'une quelconque des revendications précédentes, dans lequel le capteur de pression (30) est agencé de sorte qu'une valeur de pression de décalage est au moins obtenue lorsque le tube (T) est installé dans la zone de réception (40), le logement (50) est fermé, et le fluide ne s'écoule pas à travers le tube.

10. Système selon l'une quelconque des revendications précédentes, dans lequel la deuxième portion de contact (32) comprend une portion en saillie (32-1) qui fait saillie d'une portion voisine de la zone de réception (40), dans lequel la portion en saillie (32-1) de la deuxième portion de contact (32) est adaptée pour solliciter la portion correspondante de la deuxième section (T2) du tube (T) lorsque les parties de logement sont dans la position fermée, causant ainsi une valeur de décalage prédéterminée dans le capteur de force (30).

11. Système selon l'une quelconque des revendications précédentes, dans lequel la zone de réception (40) comprend un évidement dans lequel le capteur de pression (30) est inséré de sorte qu'une surface généralement plate du capteur de pression (30) pénètre dans la deuxième portion (40-2) de la zone de réception (40), formant ainsi la portion en saillie (32-1) .

12. Système selon l'une quelconque des revendications précédentes, dans lequel la zone de réception (40) comprend des portions de différentes formes de façon à assurer une orientation/insertion correcte du tube (T), de préférence dans lequel la première portion de contact (22) et la deuxième portion de contact (32) ont différentes formes.

13. Système selon l'une quelconque des revendications précédentes, dans lequel le capteur de pression (30) est relié à un moyen de commande adapté pour identifier la mauvaise insertion du tube (T) sur la base du signal du capteur de pression (30).

14. Tube insérable de manière amovible (T) pour un système selon l'une quelconque des revendications précédentes, comprenant une première section (T1) et une deuxième section (T2),
dans lequel la première section (T1) est configurée en tant que manchon métallique, et
dans lequel la deuxième section (T2) est une section élastique configurée pour fournir une réponse de déformation indiquant une pression de fluide, de préférence dans la plage de 10 micromètres à plusieurs 100 micromètres.

15. Appareil pour régler ou stabiliser la température d'un patient par perfusion comprenant le système selon l'une quelconque des revendications précédentes, l'appareil comprenant au moins trois unités de détection (10-1, 10-2, 10-3),
dans lequel les trois unités de détection (10) sont adaptées pour recevoir trois tubes (4a, 4b, 4c),
dans lequel l'appareil est adapté pour recevoir les trois tubes (4a, 4b, 4c) de sorte que les flux de volume (3a, 3b) de deux fluides entrant dans deux (4a, 4b) des trois tubes (4a, 4b, 4c) ont pour résultat un flux de volume commun (3c) dans le troisième (4c) des trois tubes (4a, 4b, 4c), le troisième tube (4c) étant relié à une aiguille de perfusion,
dans lequel deux (10-1, 10-2) des trois unités de détection (10-1, 10-2, 10-3) sont adaptées pour mesurer la pression et la température des flux de volume (3a, 3b) dans les deux tubes (4a, 4b) et une (10-3) des trois unités de détection (10-1, 10-2, 10-3) est adaptée pour mesurer la pression et la température du flux de volume (3c) dans le troisième tube (4c) relié avec l'aiguille de perfusion.
